**Europäisches Patentamt**

**European Patent Office**

**Office européen des brevets**

(19)

(11) Veröffentlichungsnummer: **0 544 120 B1**

(12)

# EUROPÄISCHE PATENTSCHRIFT

(45) Veröffentlichungstag der Patentschrift: **19.07.95**

(51) Int. Cl.⁶: **C07C 45/64**, C07C 47/19, B01J 27/14

(21) Anmeldenummer: **92118583.1**

(22) Anmeldetag: **30.10.92**

(54) **Verfahren zur Herstellung von 3-Hydroxyalkanalen.**

(30) Priorität: **27.11.91 DE 4138982**

(43) Veröffentlichungstag der Anmeldung:
**02.06.93 Patentblatt 93/22**

(45) Bekanntmachung des Hinweises auf die
Patenterteilung:
**19.07.95 Patentblatt 95/29**

(84) Benannte Vertragsstaaten:
**AT BE CH DE DK ES FR GB IT LI LU NL PT SE**

(56) Entgegenhaltungen:
**US-A- 3 536 763**

(73) Patentinhaber: **Degussa Aktiengesellschaft
Weissfrauenstrasse 9
D-60311 Frankturt (DE)**

(72) Erfinder: **Haas, Thomas, Dr.
Hölderlinstrasse 20
W-6000 Frankturt/Main 1 (DE)**
Erfinder: **Arntz, Dietrich, Dr.
Lorsbachstrasse 32
W-6370 Oberursel (DE)**
Erfinder: **Brand, Reinhold, Dr.
Gustav-Adolf-Strasse 25
W-6450 Hanau 1 (DE)**

## Beschreibung

Die Erfindung richtet sich auf ein Verfahren zur Herstellung von 3-Hydroxyalkanalen mit 3 bis 12 Kohlenstoffatomen, insbesondere 3-Hydroxypropionaldehyd, durch Hydratisierung der zugrundeliegenden 2-Alkenale mit Wasser in heterogener Phase in Gegenwart eines festen Katalysators mit sauren funktionellen Gruppen.

2-Alkenale der allgemeinen Formel $H_2C=CR\text{-}CHO$ mit R gleich Wasserstoff oder Alkyl, insbesondere Acrolein und Methacrolein, lassen sich gemäß US 2,434,110 in Gegenwart saurer Katalysatoren mit Wasser hydratisieren, wobei die entsprechenden 3-Hydroxyalkanale gebildet werden. Aus Acrolein ist 3-Hydroxypropionaldehyd (HPA) zugänglich, woraus durch Hydrierung das als Monomerbaustein für Polyester und Polyurethane zunehmend Bedeutung erlangende 1,3-Propandiol herstellbar ist.

Im Verfahren gemäß der US 2,434,110 werden als Katalysator im Reaktionsgemisch homogen gelöste Säuren, wie beispielsweise Schwefelsäure, Salzsäure, Phosphorsäure, Oxalsäure, saure Salze, Essigsäure, verwendet; als bevorzugt wird Schwefelsäure herausgestellt. Nachteilig an diesem Verfahren sind die niedrigen Selektivitäten.

Um die Selektivität der Hydratisierung von Acrolein zu verbessern, wurden weitere Verfahren entwickelt. Unter Verwendung von Kohlendioxid als Katalysator - siehe GB 1,185,615 - sind zwar brauchbare Selektivitäten erzielbar, aber die erforderliche lange Reaktionszeit mindert in erheblichem Umfang die Raum-Zeit-Ausbeute des Verfahrens. Schließlich können auch heterogene Katalysatoren, nämlich schwach saure, carboxylgruppenhaltige Ionenaustauscher eingesetzt werden - siehe US 3,536,763. In der Praxis zeigte sich, daß konventionelle carboxylgruppenhaltige Ionenaustauscher eine begrenzte Wirksamkeit zeigen und damit lange Reaktionszeiten erfordern. Eine Verbesserung bezüglich der Raum-Zeit-Ausbeute bei gleichzeitig hoher Selektivität erbrachten Ionenaustauscher mit Phosphonsäuregruppen - siehe DE-OS 39 26 136.

Nachteilig an den Verfahren unter Verwendung von Ionenaustauschern auf der Basis einer organischen Polymermatrix mit Phosphonatgruppen gemäß DE-A 39 26 136 sowie chelatbildenden Ankergruppen gemäß der noch unveröffentlichten DE-Patentanmeldung P 40 38 192.7 ist der Kostenaufwand für die Ionenaustauscher, die begrenzte Temperaturbeständigkeit und daß der desaktivierte Ionenaustauscher nicht regeneriert werden kann.

Die Aufgabe der vorliegenden Anmeldung besteht somit darin, weitere Katalysatoren für die heterogene Katalyse zu finden, welche leicht zugänglich sind und wie die vorerwähnten speziellen Ionenaustauscher die Hydratisierung von 2-Alkenalen, wie insbesondere Acrolein, in hoher Selektivität erlauben.

Gefunden wurde ein Verfahren zur Herstellung von 3-Hydroxyalkanalen mit 3 bis 12 C-Atomen durch Hydratisierung der zugrundeliegenden 2-Alkenale mit Wasser in heterogener Phase in Gegenwart eines festen Katalysators mit sauren funktionellen Gruppen bei einer Reaktionstemperatur von 20 °C bis 120 °C, einem Druck von 1 bar bis 20 bar und einer Anfangskonzentration des 2-Alkenals im flüssigen Reaktionsgemisch von 3 bis 30 Gew.-%, das dadurch gekennzeichnet ist, daß der verwendete Katalysator aufgebaut ist aus einem anorganischen Träger mit basischen Aktivitätszentren, welche mindestens teilweise mit einer mehrwertigen Säure, deren erster $pk_s$-Wert zwischen 0 und 3 liegt, in durch Wasser nicht ablösbarer Form belegt sind.

Die Trägermaterialien müssen eine ausreichende Stabilität gegenüber der aufzubringenden Säure aufweisen. Geeignete Trägermaterialien sind solche, die sowohl saure als auch basische Zentren besitzen, insbesondere Oxide und Mischoxide. Das Verhältnis von Zahl und Stärke der sauren bzw. basischen Zentren sollte so sein, daß eine 10 gew.-%ige wäßrige Aufschlämmung des Trägerpulvers in Wasser zu einem pH-Wert von 2 bis 5, bevorzugt 3 bis 4, führt. Pyrogenes $TiO_2$ wird besonders bevorzugt, da es sowohl saure als auch basische Zentren in ausgewogenem Verhältnis besitzt (K.I. Khadzhiivanöv, A.A. Davydov, D.G. Klisurski; Kinetika i Kataliz 29, 1, 161-167) und der entsprechende pH bei 3,5 liegt. Weniger geeignet sind stark saure (z. B. Al-silikate) bzw. stark basische Oxide (z. B. $\gamma\text{-}Al_2O_3$).

Als besonders geeignete mehrwertige Säure, deren $pK_s$-Wert der ersten Dissoziationsstufe im beanspruchten Bereich, nämlich bei +1,96, liegt, ist Phosphorsäure.

Die auf dem anorganischen Träger fixierte mehrwertige Säure darf während der Hydratisierung praktisch nicht abgelöst werden, weil ansonsten im Rahmen der Weiterverarbeitung des 3-Hydroxyalkanal enthaltenden Reaktionsgemisches gegebenenfalls eine Reinigungsstufe erforderlich würde und zudem die Standzeit des Katalysators gemindert würde.

Träger und mehrwertige Säure werden so aufeinander abgestimmt, daß einerseits eine ausreichende Acidität an der Oberfläche des Katalysators resultiert, andererseits das System als Säure-Base-Puffer wirkt. Der Fachmann wird die erforderliche Abstimmung zwischen Träger und Säure durch einem orientierenden Versuch ermitteln.

Die erfindungsgemäßen Katalysatoren lassen sich durch eine übliche Imprägnierung des anorganischen Trägers mit einer wäßrigen Lösung der mehrwertigen Säure, Trocknen, Kalzinieren bei oberhalb 150 °C, insbesondere bei 250 bis 500 °C, und Ausspülen mit Wasser bis zur Säurefreiheit des Waschwassers herstellen. Es wird angenommen, daß bei der Kalzinierung zwischen dem Träger und dem Zentralatom der Säure eine feste Bindung über ein Sauerstoffatom hergestellt wird - beispielsweise Ti-O-P im Falle des besonders bevorzugten Katalysators auf der Basis $TiO_2/H_3PO_4$.

Als mehrwertige Säuren kommen insbesondere Oxosäuren der Elemente Phosphor, Arsen, Vanadin, Chrom, Molybdän und Wolfram infrage, wobei es sich auch um Homopolysäuren oder Heteropolysäuren der genannten Elemente handeln kann. Säuren mit einem $pK_s$-Wert für die erste Dissoziationsstufe unter Null werden ausgeschlossen, weil sie zu Katalysatoren mit zu geringer Selektivität führen. Säuren mit einem ersten $pK_s$-Wert oberhalb 3 lassen sich zwar noch an den Trägern fixieren, jedoch sinkt auch hier die Selektivität, und das 2-Alkenal polymerisiert gegebenenfalls auf dem Katalysator.

2-Alkenal, insbesondere Acrolein, und Wasser werden als Gemisch in Gegenwart des heterogenen Katalysators umgesetzt, wobei die Temperatur so eingestellt wird, daß die flüssige Phase homogen bleibt. Die Startkonzentration an 2-Alkenal in der flüssigen Phase liegt zwischen 3 und 30 Gew.-%, insbesondere zwischen 10 und 18 Gew.-%. Sofern erwünscht, können dem 2-Alkenal-Wasser-Gemisch Polymerisationsinhibitoren, wie z. B. Hydrochinon, Hydrochinonmonomethylether oder butylierte Phenole, in wirksamer Menge zugegeben werden. Bei Kreislaufführung der flüssigen Phase über ein Katalysatorbett enthält die flüssige Phase auch gebildetes 3-Hydroxyalkanal und Nebenprodukte aus der Umsetzung des 2-Alkenals bzw. Folgeprodukte des gebildeten 3-Hydroxyalkanals. Die Hydratisierung kann in einem weiten Temperaturbereich erfolgen. Die Reaktionstemperatur liegt vorzugsweise bei 50 °C bis 90 °C. Üblicherweise wird unter Normaldruck oder nur mäßigem Überdruck gearbeitet.

Die Hydratisierung kann diskontinuierlich oder kontinuierlich durchgeführt werden, wobei an sich bekannte Reaktoren, wie beispielsweise Rührreaktoren, Schlaufenreaktoren, Schwebebett-, Wirbelbett- und Strömungsrohrreaktoren, einsetzbar sind. Nach dem Strömungsrohrprinzip arbeitende Reaktoren werden gegenüber Schlaufen- und Rührreaktoren bevorzugt. Die Durchflußgeschwindigkeit durch ein Strömungsrohr, das den erfindungsgemäßen heterogenen sauren Katalysator enthält und mit einem heizbaren Mantel versehen ist, sowie die Reaktionstemperatur wird man so einstellen, daß der gewünschte Acrolein-Umsatz erreicht wird. Üblicherweise beträgt der LHSV-Wert (liquid hourly space velocity) 0,1 bis 2 $h^{-1}$, vorzugsweise mehr als 0,5 $h^{-1}$. Die Reaktionsmischung kann auch mehrfach über das Katalysatorbett geführt werden, sofern größere Totvolumina vermieden werden, in welchen die Reaktionsmischung bei Reaktionstemperatur in Abwesenheit des Hydratisierungskatalysators vorliegt.

Nach der Abtrennung des heterogenen Katalysators, was üblicherweise durch Sedimentation oder Filtration erfolgt oder sich bei Verwendung von an sich bekannten Einrichtungen für Festbettkatalysatoren praktisch von alleine ergibt, wird das Reaktionsgemisch, soweit erforderlich, von nicht-umgesetztem Acrolein befreit.

Bei einem Acrolein-Umsatz zwischen 30 bis 80 % wird diese Maßnahme stets empfehlenswert sein, weil Acrolein, auch wenn dieses als wasserhaltiges Acrolein abgetrennt worden ist, direkt in den Prozeß zurückgeführt werden kann. Die Abtrennung des Acroleins kann in bekannter Weise destillativ, vorzugsweise unter vermindertem Druck und Temperaturen unter 80 °C, verwirklicht werden. Besonders günstig ist es, das Reaktionsgemisch einem Dünnschichtverdampfer zuzuführen, wobei unter schonenden Bedingungen sowohl nicht-umgesetztes Acrolein als auch ein Teil des Wassers des Reaktionsgemisches gemeinsam abdestilliert werden. Auf diese Weise erhält man als Sumpfprodukt eine konzentrierte wäßrige Lösung von 3-Hydroxyalkanal, was sich vorteilhaft auf die Weiterverarbeitung des Reaktionsgemischs und den Energieverbrauch des Verfahrens auswirkt.

Wie bereits angedeutet, wird das 3-Hydroxyalkanal enthaltende Reaktionsgemisch nach Abtrennung des nicht umgesetzten 2-Alkenals im allgemeinen als solches der Umsetzung zu Folgeprodukten wie insbesondere der Hydrierung zugeführt, ohne daß das 3-Hydroxyalkanal zuvor isoliert wird.

Die katalytische Hydrierung von beispielsweise 3-Hydroxypropionaldehyd zu 1,3-Propandiol in flüssiger Phase wird in an sich bekannter Weise und in üblichen Hydrierapparaturen durchgeführt. Der Katalysator kann sowohl in suspendierter Form per se oder trägergebunden vorliegen oder Bestandteil von Festbettkatalysatoren sein; auch homogene Katalysatoren können herangezogen werden. Als Suspensionskatalysatoren sind Raney-Nickel, das mit verschiedenen anderen katalytisch wirksamen Metallen dotiert sein kann, sowie Platin auf Aktivkohle und anderen Trägern besonders geeignet. Unter den Festbettkatalysatoren kommen z. B. die in der US-PS 2,434,110 genannten Stoffe zur Anwendung; Nickelkatalysatoren haben sich als besonders wirkungsvolle Katalysatoren erwiesen.

Eine hohe Raum-Zeit-Ausbeute wird in der Hydrierstufe erzielt, wenn die zu hydrierende Lösung einen pH-Wert im Bereich von 2,5 bis 6,5 , vorzugsweise um 6, aufweist und die Hydriertemperatur im Bereich

3

zwischen 25 und 100 °C liegt.

Mit den neuen Katalysatoren wurde die Palette einsatzfähiger Hydratisierungskatalysatoren erweitert. Selektivitäten oberhalb 70 % lassen sich damit leicht erreichen. Die Träger der neuen Katalysatoren sowie die mehrwertigen Säuren sind leicht zugänglich, und die Herstellung der Katalysatoren bereitet keine Schwierigkeit. Es war nicht vorhersehbar, daß die gefundenen Katalysatoren die erforderliche Wirksamkeit zeigen und eine hohe Standzeit aufweisen, was sich günstig auf die Wirtschaftlichkeit der Hydratisierung der 2-Alkenale auswirkt.

Gegenüber der Verwendung saurer Ionenaustauscher auf der Basis einer organischen Polymermatrix als heterogene Katalysatoren können die erfindungsgemäßen Katalysatoren auf der Basis anorganischer Träger bei höheren Reaktionstemperaturen ohne Gefahr einer irreversiblen Schädigung betrieben werden. Durch höhere Reaktionstemperaturen können die Katalysator-und/oder Reaktorkosten reduziert werden. Außerdem können desaktivierte Katalysatoren auf Basis anorganischer Träger regeneriert werden, etwa durch Abbrennen oder Oxidation der kohlenstoffhaltigen Ablagerungen und, falls erforderlich, erneuter Belegung mit der mehrwertigen Säure, was mit Katalysatoren auf Basis einer organischen Polymermatrix nicht möglich ist.

**Beispiele 1-2 und Vergleichsbeispiele VB1-VB2**

Zur Bestimmung der Aktivität und Selektivität der Katalysatoren der Erfindung wird folgender Test durchgeführt:

Ein 50 ml-Septumfläschen wird mit 25 ml Katalysator befüllt und soviel Wasser zugegeben, daß der Katalysator gerade bedeckt ist. Zusätzlich werden 1,6 g Wasser und Acrolein (Ac) zugegeben, so daß sich eine Ac-konzentration in der wäßrigen Phase von etwa 18 Gew.-% einstellt. Die Mischung wird 3 Minuten bei Raumtemperatur durch rotierende Bewegung geschüttelt und die aktuelle Acrolein-Konzentration einer Probe gaschromatographisch bestimmt. Die Probe wird für eine bestimmte Reaktionsdauer im Wasserbad bei der angegebenen Temperatur weitergeschüttelt und dann analysiert. Der Tabelle 1 sind Angaben zum Träger und der darauf fixierten Säure, der Umsatz, die Selektivität nach der angegebenen Reaktionsdauer, die Startkonzentration an Acrolein und die Reaktionstemperatur zu entnehmen.

Tabelle

| Beispiel-Nr. bzw. Vergleichs-beispiel-Nr. | Katalysator | | Acrolein-Start-konzentration (Gew.-%) | Reaktions-temp. (°C) | Hydratisierung | | |
|---|---|---|---|---|---|---|---|
| | Träger | Säure | | | Reaktions-dauer (h) | Umsatz (%) | Selekt. (%) |
| 1 | TiO$_2$ | H$_3$PO$_4$ | 18,5 | 70 | 1 | 42 | 81 |
| VB1 | TiO$_2$ | NaH$_2$PO$_4$ | 19,0 | 70 | 1 | 41 | 50 |
| 2 | g-Al$_2$O$_3$ | H$_3$PO$_4$ | 17,0 | 50 | 6,5 | 58 | 71,4 |
| VB2 | g-Al$_2$O$_3$ | NaH$_2$PO$_4$ | 16,0 | 70 | 1 | 91 | 3 |

**Beispiel 3**

In einer Laborapparatur mit einem Festbettreaktor mit phosphorsäureimprägniertem TiO$_2$ als Katalysator wird die Hydratisierung von Acrolein kontinuierlich über einen längeren Zeitraum durchgeführt. Umsatz und Selektivität werden durch Analyse der Produktlösung bestimmt.

5

Die Apparatur besteht aus einem skalierten, kühlbaren 2 l-Glasgefäß für die wäßrige Acrolein-Ausgangslösung, einer HPLC-Pumpe für die Förderung der Reaktionsmischung, einem thermostatierten, druckbeständigen, präzisionsgeschliffenen Glasrohr (300 mm x 26 mm i.D.), das die Katalysatorfüllung aufnimmt und an beiden Enden mit verstellbaren Verschraubungen verschlossen ist, einem Druckhalteventil, sowie einer auf +5 °C gekühlten 2 l-Glasvorlage für die Produktlösung und den notwendigen Thermostatisiereinrichtungen.

Die Acrolein-Wasser-Mischung wird vorgelegt und mittels der HPLC-Pumpe durch das auf Reaktionstemperatur gehaltene Festbett aus 138 ml Katalysator bei konstantem Volumenstrom gepumpt. Nach einer Einfahrzeit von 3-5 h zur Einstellung des stationären Zustands wird nach Wechseln der Vorlage unter konstanten Versuchsbedingungen über einen Zeitraum von mehreren hundert Stunden Acroleinlösung durch das thermostatisierte Glasrohr gepumpt. In bestimmten zeitlichen Abständen (etwa alle 48 h) wird die Produktlösung analysiert.

| | |
|---|---|
| Eingesetzter Katalysator: | Phosphorsäureimprägniertes $TiO_2$ (gemäß Beispiel 5) |
| Acrolein-Startkonzentration: | 16,5 Gew.-% |
| Reaktionstemperatur: | 50-70 °C |
| LHSV (= liquid hourly space velocity): | 0,5 $h^{-1}$ |

Die gesamte Versuchsdauer betrug 900 h. Während dieser Zeit wurde der Umsatz bei 50 % ± 2 % gehalten. Die Temperatur wurde im Laufe des Versuchs von 50 °C auf 70 °C angehoben, um den Umsatz konstant zu halten. Die Selektivität der Reaktion betrug 81 % ± 2 %.

Nach 900 h Versuchsdauer konnte in der erhaltenen Produktlösung kein Phosphor nachgewiesen werden (Nachweisgrenze 1 mg/l); der Phosphorgehalt des Katalysators betrug nach 900 h 2000 ppm.

Nach dem 900 h-Versuch wurde die Reaktionstemperatur auf 100 °C angehoben und der Versuch bei einem LHSV-Wert von 0,5 $h^{-1}$ fortgeführt. Auch unter diesen Bedingungen lag der Phosphorgehalt in der Lösung unterhalb der Nachweisgrenze.

**Beispiel 4**

Hydrierung einer gemäß Beispiel 3 erhaltenen wäßrigen Lösung von 3-Hydroxypropionaldeyd (HPA)

Die Reaktionslösung des Beispiels 3 wird über eine Destillationskolonne bei 500 mbar von nicht umgesetztem Acrolein befreit. 500 g der 8,7 %igen HPA-Lösung werden in einem 1000 ml-Autoklaven mit Begasungsrührer bei 135 bar Wasserstoffdruck, einer Reaktionstemperatur von 60 °C und einer Rührerdrehzahl von 1000 UpM in Gegenwat von 5 g Raney-Nickel bei pH 4 innerhalb 2 h hydriert. Der HPA-Umsatz betrug 99,9 % und die Ausbeute an 1,3- Propandiol, bezogen auf eingesetztes HPA, betrug 99,8 %.

**Beispiel 5**

Herstellung der in den Beispielen 1 und 2 sowie Vergleichsbeispielen VB1 und VB2 eingesetzten Katalysatoren:

200 g Träger - gamma-Aluminiumoxid beziehungsweise pyrogen hergestelltes Titanoxid mit einer spezifischen Oberfläche (BET) von 50 $m^2/g$ - werden in 20 gew.-%iger wäßriger $H_3PO_4$ - beziehungsweise $NaH_2PO_4$-Lösung 12 h stehen gelassen. Die so imprägnierten Träger werden 4 h bei 150 °C getrocknet und anschließend 1 h bei 450 °C kalziniert. Die so hergestellten Katalysatoren werden bei 80 °C mit Wasser bis zur Säurefreiheit des Waschwassers gewaschen.

**Patentansprüche**

**1.** Verfahren zur Herstellung von 3-Hydroxyalkanalen mit 3 bis 12 C-Atomen durch Hydratisierung der zugrundeliegenden 2-Alkenale mit Wasser in heterogener Phase in Gegenwart eines festen Katalysators mit sauren funktionellen Gruppen bei einer Reaktionstemperatur von 20 °C bis 120 °C, einem Druck von 1 bar bis 20 bar und einer Anfangskonzentration des 2-Alkenals im flüssigen Reaktionsgemisch von 3 bis 30 Gew.-%,
dadurch gekennzeichnet,
daß der verwendete Katalysator aufgebaut ist aus einem anorganischen Träger mit basischen Aktivitätszentren, welche mindestens teilweise mit einer mehrwertigen Säure, deren erster pk$_s$-Wert zwischen 0 und 3 liegt, in durch Wasser nicht ablösbarer Form belegt sind.

2.  Verfahren nach Anspruch 1,
    dadurch gekennzeichnet,
    daß der anorganische Träger im wesentlichen aus Titandioxid, vorzugsweise pyrogen hergestelltem Titandioxid, besteht.

3.  Verfahren nach Anspruch 1 oder 2,
    dadurch gekennzeichnet,
    daß als mehrwertige Säure Phosphorsäure auf dem Träger fixiert ist.

4.  Verfahren nach Anspruch 1 bis 3,
    dadurch gekennzeichnet,
    daß der Katalysator hergestellt worden ist durch Imprägnieren eines oxidischen Trägers, vorzugsweise TiO$_2$, mit Phosphorsäure, Kalzinieren des getrockneten Produkts bei 250 °C bis 500 °C und Auswaschen des kalzinierten Produktes mit Wasser bis zur Säurefreiheit desselben.

5.  Verfahren nach einem oder mehreren der Ansprüche 1 bis 4,
    dadurch gekennzeichnet,
    daß man als 2-Alkenal Acrolein einsetzt.

6.  Verfahren nach einem oder mehreren der Ansprüche 1 bis 5,
    dadurch gekennzeichnet,
    daß die Reaktionstemperatur 50 °C bis 90 °C beträgt.

7.  Verfahren nach einem oder mehreren der Ansprüche 1 bis 6,
    dadurch gekennzeichnet,
    daß man die Startkonzentration an 2-Alkenal im flüssigen Reaktionsgemisch auf 6 bis 20 Gew.-%, vorzugsweise 10 bis 18 Gew.-%, einstellt.

8.  Verfahren nach einem oder mehreren der Ansprüche 1 bis 7,
    dadurch gekennzeichnet,
    daß man den Katalysator in einem Festbett anordnet und die Hydratisierung durch Überleiten des wäßrigen, 2-Alkenals enthaltenden Reaktionsgemischs bei LHSV-Werten zwischen 0,6 und 1,5 h$^{-1}$ durchführt.

**Claims**

1.  A process for the production of 3-hydroxyalkanals containing 3 to 12 carbon atoms by hydration of the basic 2-alkenals with water in heterogeneous phase in the presence of a solid catalyst containing acidic functional groups at a reaction temperature of 20°C to 120°C, under a pressure of 1 bar to 20 bar and with an initial concentration of the 2-alkenal in the liquid reaction mixture of 3 to 30% by weight, characterized in that the catalyst used is made up of an inorganic support having basic activity centers which are at least partly occupied by a polybasic acid, of which the first pK$_s$ value is between 0 and 3, in a form in which it cannot be removed by water.

2.  A process as claimed in claim 1, characterized in that the inorganic support consists essentially of titanium dioxide, preferably pyrogenically produced titanium dioxide.

3.  A process as claimed in claim 1 or 2, characterized in that phosphoric acid is fixed to the support as the polybasic acid.

4.  A process as claimed in claims 1 to 3, characterized in that the catalyst has been produced by impregnation of an oxidic support, preferably TiO$_2$, with phosphoric acid, calcination of the dried product of 250 to 500°C and washing of the calcined product with water until the washing water is acid-free.

**5.** A process as claimed in one or more of claims 1 to 4, characterized in that acrolein is used as the 2-alkenal.

**6.** A process as claimed in one or more of claims 1 to 5, characterized in that the reaction temperature is in the range from 50°C to 90°C.

**7.** A process as claimed in one or more of claims 1 to 6, characterized in that the starting concentration of 2-alkenal in the liquid reaction mixture is adjusted to between 6 and 20% by weight and preferably to between 10 and 18% by weight.

**8.** A process as claimed in one or more of claims 1 to 7, characterized in that the catalyst is arranged in a fixed bed and the hydration is carried out by passing the aqueous reaction mixture containing 2-alkenal over the catalyst at LHSV values of 0.6 to 1.5 h$^{-1}$.

**Revendications**

**1.** Procédé de préparation de 3-hydroxyalcanals avec de 3 à 12 atomes de C par hydratation des 2-alcénals pris pour base avec de l'eau en phase hétérogène en présence d'un catalyseur fixe avec des groupes acides fonctionnels à une température de réaction de 20°C à 120°C, une pression de 1 bar à 20 bars et une concentration de débit du 2-alcénal dans le mélange réactionnel liquide de 3 à 30 % en poids, caractérisé en ce que le catalyseur utilisé est constitué à partir d'un support minéral avec des centres d'activité basiques, qui sont recouverts sous une forme non détachable par l'eau, d'un acide plurivalent, dont la première valeur pk$_s$ se situe entre 0 et 3.

**2.** Procédé selon la revendication 1, caractérisé en ce que le support minéral est composé essentiellement de dioxyde de titane, de préférence de dioxyde de titane préparé par pyrogénération.

**3.** Procédé selon les revendications 1 ou 2, caractérisé en ce que l'acide phosphorique en tant qu'acide plurivalent est fixé sur le support.

**4.** Procédé selon les revendications 1 à 3, caractérisé en ce que le catalyseur a été préparé par imprégnation d'un support obtenu par voie d'oxydation, de préférence TiO$_2$, avec de l'acide phosphorique , calcination du produit séché de 250°C à 500°C et lavage du produit calciné à l'eau jusqu'à l'élimination de l'acide de celui-ci même.

**5.** Procédé selon une ou plusieurs des revendications 1 à 4, caractérisé en ce qu'on met en oeuvre de l'acroléine comme 2-alcénol.

**6.** Procédé selon une ou plusieurs des revendications 1 à 5, caractérisé en ce que la température réactionnelle atteint 50°C à 90°C.

**7.** Procédé selon une ou plusieurs des revendications 1 à 6, caractérisé en ce qu'on ajuste la concentration de départ en 2-alcénal dans le mélange réactionnel liquide de 6 à 20 % en poids, de préférence de 10 à 18 % en poids.

**8.** Procédé selon une ou plusieurs des revendications 1 à 7, caractérisé en ce qu'on dispose le catalyseur sur un lit solide et on réalise l'hydratation par passage du mélange réactionnel aqueux, contenant du 2-alcénal, avec des valeurs LHSV comprises entre 0,6 et 1,5$^{-1}$.